(19) Europäisches Patentamt
European Patent Office
Office européen des brevets

(11) Veröffentlichungsnummer: **0 101 905**
**B1**

(12) **EUROPÄISCHE PATENTSCHRIFT**

(45) Veröffentlichungstag der Patentschrift: **10.06.87**

(51) Int. Cl.⁴: **C 08 K 5/06**, C 08 L 21/00, C 07 C 43/16

(21) Anmeldenummer: **83107183.2**

(22) Anmeldetag: **22.07.83**

(54) **Verfahren zur Verbesserung der Ozonbeständigkeit von Kautschuk und dafür geeignete Enolether.**

(30) Priorität: **03.08.82 DE 3228864**

(43) Veröffentlichungstag der Anmeldung: **07.03.84 Patentblatt 84/10**

(45) Bekanntmachung des Hinweises auf die Patenterteilung: **10.06.87 Patentblatt 87/24**

(84) Benannte Vertragsstaaten: **DE FR GB IT**

(56) Entgegenhaltungen:
**DE-A-1 917 600**
**US-A-1 959 927**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Brück, Dieter Wolfram, Dr., Kattowitzer-Strasse 34, D-5000 Köln 80 (DE)**
Erfinder: **Jeblick, Werner, Dr., Walter- Flex-Strasse 30, D-5090 Leverkusen 1 (DE)**
Erfinder: **Ruetz, Lothar, Dr., Wilhelm- Busch-Strasse 37, D-4047 Dormagen 5 (DE)**

**EP 0 101 905 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein Verfahren zur Verbesserung der Ozonbeständigkeit von Natur- und/oder Synthesekautschuk mittels Enolether der allgemeinen Formel (I)

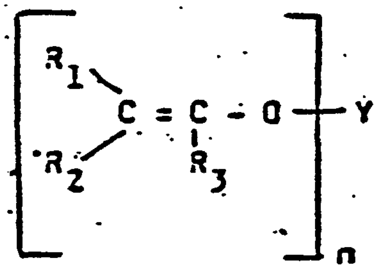

(I)

in der
n 1 oder 2,
$R^1$ $C_1$-$C_{10}$-Alkyl oder $C_2$-$C_{10}$-Alkenyl, welche gegebenenfalls ein- oder mehrfach, bevorzugt ein- bis dreifach durch Chlor oder $C_1$-$C_4$-Alkyl substituiert sind, $C_5$-$C_{20}$-Cyclo- oder Bicycloalkyl oder -alkenyl; $C_6$-$C_{10}$-Aryl, gegebenenfalls ein- oder mehrfach, bevorzugt ein- bis fünffach durch Chlor und/oder $C_1$-$C_4$-Alkyl substituiert, oder $C_6$-$C_{10}$-Aryl-$C_1$-$C_8$-alkyl, wobei die Arylgruppe ein- oder mehrfach, bevorzugt ein- bis fünffach durch Halogen und/oder $C_1$-$C_4$-Alkyl substituiert sein kann, bedeutet, $R^2$ und $R^3$ Wasserstoff bedeuten oder die Bedeutung von $R^1$ aufweisen und
die beiden Substituenten $R_1$ und $R_3$ zu einem mindestens fünfgliedrigen Ring miteinander verbunden sein können, und
Y im Fall von n = 1
$C_5$-$C_7$-Cycloalkyl, $C_5$-$C_7$-Bicycloalkyl, $C_5$-$C_7$-Cycloalkenyl, $C_5$-$C_7$-Bicycloalkenyl, $C_6$-$C_{10}$-Aryl, $C_5$-$C_7$-Cycloalkyl-$C_1$-$C_4$-alkyl, $C_5$-$C_7$-Cycloalkenyl-$C_1$-$C_4$-alkyl, $C_5$-$C_7$-Bicycloalkenyl-$C_1$-$C_4$-alkyl oder gegebenenfalls durch ein bis drei Sauerstoff- oder durch $R_4$ substituierte Stickstoffatome unterbrochenes $C_4$-$C_{12}$-Alkyl und $C_4$-$C_{12}$-Alkenyl,
im Fall n = 2
$C_5$-$C_7$ Cycloalkylen, $C_5$-$C_{10}$ Arylen, Xylylen, Hexahydroxylylen oder gegebenenfalls durch ein bis drei Sauerstoff- oder durch $R_4$ substituierte Stickstoffatome unterbrochenes $C_4$-$C_{12}$-Alkylen darstellt und wobei alle Reste Y gegebenenfalls ein- oder mehrfach, bevorzugt ein bis vierfach durch Chlor substituiert sind und $R_4$ $C_1$-$C_4$-Alkyl bedeutet.

Unter den Begriffen Alkyl, Alkylen oder Alkenyl werden sowohl geradkettige wie verzweigte Reste verstanden.

Bevorzugt sind
$R_1$ $C_1$-$C_6$-Alkyl,
$R_2$ Wasserstoff, Methyl oder Ethyl, oder $R_1$ und $R_2$ zusammen Pentamethylen,
$R_3$ Wasserstoff und
Y Tetramethylen, Hexamethylen, Di-Hexamethylenether, Xylylen, 1,4-Dimethylencyclohexylen, $C_4$-$C_{10}$-Alkyl, $C_5$-$C_7$-Cycloalkenylmethyl, $C_5$-$C_7$-Bicycloalkenylmethyl.

Die Verbindungen der Formel I können durch Acetalisierung eines Aldehyds oder Ketons mit der doppelt äquivalenten Menge einer OH-Verbindung und daran anschließender Abspaltung eines Äquivalents der OH-Verbindung nach literaturbekannten Verfahren hergestellt werden; sie sind teilweise literaturbekannt.

Die Verbindnungen der Formel I sind feste oder flüssige Substanzen, welche sich gut und homogen im Rohkautschuk einarbeiten und verteilen lassen. Sie besitzen gegenüber bekannten Ozonschutzmitteln (DE-AS 1.917.600, DE-OS-2.548.911, DE-AS 1.693.163) insbesondere im Naturkautschuk eine bessere Wirksamkeit, und verfärben nicht.

Ein weiterer Gegenstand der Erfindung sind Verbindungen der Formel

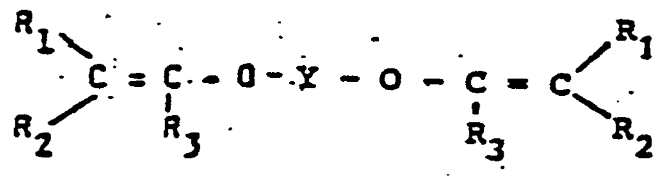

worin $R_1$, $R_2$ und $R_3$ die oben genannten Bedeutungen besitzen und

Y die Bedeutungen $C_5$-$C_7$-Cycloalkylen, $C_6$-$C_{10}$-Arylen, Xylylen oder gegebenenfalls durch ein bis drei Sauerstoffatome oder $NR_4$-Gruppen unterbrochenes $C_4$-$C_{12}$-Alkylen hat und gegebenenfalls ein- oder mehrfach durch Chlor substituiert sein kann und

$R_4$ $C_1$-$C_6$-Alkyl bedeutet.

Bevorzugte Verbindungen sind solche, in denen

$R_1$ $C_1$-$C_6$-Alkyl

$R_2$ Wasserstoff, Methyl oder Ethyl oder $R_1$ und $R_2$ zusammen Pentamethylen,

$R_3$ Wasserstoff und

Y Tetramethylen, Hexamethylen, Di-hexamethylenether, Xylylen oder 1,4-Dimethylencyclohexylen bedeuten.

Geeignete Kautschuke sind Naturkautschuk oder Synthesekautschuk, z. B. Uni- oder Mischpolymerisate konjugierter Diolefine wie Butadien, Dimethylbutadien, Chloropren und Isopren mit Vinylverbindungen wie Styrol, -Methylstyrol, Acrylnitril oder Methacrylnitril, Poly-(meth)acrylate, sowie Terpolymere aus Ethylen, Propylen mit mindestens einem nichtkonjugierten Dien wie Dicyclopentadien, 5-Ethyliden-2-norbornen oder 1,4 Hexadien.

Die Ozonschutzmittel können in die Kautschuke auf übliche Weise eingemischt werden, beispielsweise auf einem Mischwalzwerk oder im Innenmischer. Sie werden vor oder gleichzeitig mit den anderen Bestandteilen zugesetzt; können aber auch als letzter Mischungsbestandteil zugegeben werden.

Übliche weitere Bestandteile sind beispielsweise Vulkanisiermittel wie Schwefel, Vukanisationsbeschleuniger, beispielsweise Thiazole wie 2-Merkaptobenzthiazol, Dibenzothiazyldisulfid, Sulfenamide wie Benzothiazyl-2-cyclohexyl-sulfenamid, Benzothiazyl-2-butylsulfenamid oder Benzothiazylsulfensäuremorpholid, Guanidine wie Diphenylguanidin oder Di-o-tolylguanidin, Dithiocarbamate wie Zink-diethyldithiocarbamat, Thiurame wie Tetramethylthiuramdisulfid und Ethylenthioharnstoff. Weiterhin können andere Hilfsstoffe, beispielsweise Füllstoffe wie Ruß oder Kreide, Anitoxidantien, Wachse, Pigmente, Zinkoxid, Stearinsäure und Verarbeitungsöle zugemischt werden.

Die Verbindungen der Formel I werden bevorzugt in Mengen von 0,05 bis 10 Gew.%, insbesondere 0,5 bis 5 Gew.%, bezogen auf Kautschuk, eingemischt.

Die Vulkanisation des Kautschuks erfolgt durch Erhitzen auf die üblichen Temperaturen, vorzugsweise 120-170°C.

**Beispiel**

1,6-Bis-(2-methyl-prop-1-enoxy)-hexan im Gemisch mit 6,5'-Bis(2-methyl-prop-1-enoxy)-dihexylether

In einem Kolben mit Wasserabscheider werden je Mol Isobutyraldehyd, 1 Mol 1,6-Hexandiol, 1,4 g p-Toluolsulfonsäure, 0,4 g Chinolin und 400 ml Cyclohexan vorgelegt und 6,5 h auf Rückflußtemperatur erhitzt. Dabei scheiden sich je Mol Aldehyd 17 ml Wasser ab. Nach Abkühlung und Wäsche der Reaktionslösung mit 5 gew-%iger $K_2CO_3$-Lösung bzw. Wasser erhält man (n. IR-Analyse) das gewünschte Acetal nach Abzug von Cyclohexan als Rückstand. Zur Herstellung des Enolethers werden in einem Kolben mit Destillationsaufsatz (30 cm Raschigringe) je 100 Teile des Acetals, 1,5 Teile Phosphorsäure und 3,6 Teile pyridin vorgelegt, auf 150°C erwärmt und 30 Minuten bei 150°C gerührt. Anschließend wird nach Abnahme eines Vorlaufs (Hexandiol) bei ca. 0,9 mbar und ca. 85°C Kopftemperatur der Enolether zusammen mit noch verbliebenem 1,6-Hexandiol überdestilliert. Zur Abtrennung des 1,6-Hexandiols wird das Destillat in heißem Hexan aufgenommen und die erhaltene Lösung anschließend bis auf 5°C abgekühlt. Hexandiol kristallisiert aus. Aus der Mutterlauge erhält man nach Abzug des Hexans das Gemisch der beiden Titelsubstanzen.

**Beispiel 2**

1,6-Bis-(2-ethyl-hex-1-enoxy)-hexan im Gemisch mit 6,6' Bis(2-ethyl-hex-1-enoxy)-dihexylether

Die Acetalisierung von 2-Ethylhexanal-1 mit 1,6-Hexandiol und die anschließende Spaltung des Acetals zum Enolether wurde wie in Beispiel 1 durchgeführt. Restliches Hexandiol und der Enolether wurden bei ca. 0,15 mbar 145°C Kopftemperatur überdestilliert. Die Reinigung des Produktes erfolgte wie in Beispiel 1 durch Auskristallisieren des Hexandiols aus einer Hexanlösung des Destillats. Nach NMR- und IR-spektroskopischer Analyse besteht das erhaltene Produkt aus einem Gemisch der beiden Titelsubstanzen.

**Beispiel 3**

1,6-Bis-(but-1-enoxy)-hexan im Gemisch mit 6,6'-Bis-(but-1-enoxy)-dihexylether

Die Acetalisierung von 1 Mol Butyraldehyd mit 1 Mol Hexandiol-1,6 und die daran anschließende Herstellung des Enolethers wurde wie in Beispiel 1 beschrieben durchgeführt. Nach Abnahme eines Vorlaufs (Hexandiol-1,6) erhält man das gewünschte Substanzgemisch zusammen mit etwas Hexandiol-1,6 bei 65 - 70°C/0,05 - 0,1 Torr als Destillat.

Die Abtrennung des restlichen Hexandiols-1,6 erfolgt wie im Beispiel 1 beschrieben aus einer Hexanlösung des Destillats. Man erhält ein Gemisch der beiden Titelsubstanzen.

**Beispiel 4**

1,6-Bis (cyclohex-1-enoxy)-hexan

Die Acetalisierung von 1 Mol Cyclohexanon mit 1 Mol 1,6-Hexandiol und die daran anschließende Herstellung des Enolethers sowie die Aufarbeitung desselben erfolgte wie in Beispiel 1 beschrieben. Nach Abzug des Hexans wurde das resultierende Öl destilliert. Das gewünschte produkt (IR, NMR Analyse) destilliert bei 96 - 104°C/0,08 Torr.

**Beispiel 5**

1,6-Bis (2-ethyl-but-1-enoxy)-hexan.

Die Acetalisierung von 1 Mol 2-Ethylbutyraldehyd mit 1 Mol Hexandiol-1,6 und die daran anschließende Herstellung des Enolethers wurde wie in Beispiel 1 beschrieben durchgeführt. Die zur Spaltung des Acetats eingesetzte Phosphorsäure wurde jedoch zuvor azeotrop entwässert. Nach Abnahme eines Vorlaufs (Hexandiol-1,6) erhält man das gewünschte Produkt zusammen mit etwas Hexandiol-1,6 bei 92-112°C/0,1-0,2 Torr. Die Abtrennung des restlichen Hexandiols-1,6 erfolgte wie in Beispiel 1 beschrieben aus der Hexanlösung des Destillats.

**Beispiel 6**

2-Methylprop-1-enylhexylether

Die Acetalisierung von 1 Mol Isobutyraldehyd mit 2 Mol Hexanol und die daran anschließende Herstellung des Enolethers wurde wie in Beispiel 1 beschrieben durchgeführt. Das gewünschte Produkt destilliert nach Spaltung des Acetals bei ca. 88-93°C/12 Torr über.

**Beispiel 7**

(2-Ethylbut-1 enyl)-(norborn-3-en-1-methyl)-ether.

Die Acetalisierung von 0,5 Mol Ethylbutyraldehyd mit 1 Mol Norborn-3-en-1-methanol und die daran anschließende Herstellung des Enolethers wurde wie in Beispiel 1 beschrieben durchgeführt. Nach Abnahme eines Vorlaufs erhält man das gewünschte Produkt als bei 123-127°C/12 Torr übergehende Fraktion.

**Beispiel 8**

(2-Methylbut-1-enyl)-(norborn-3-en-1-methyl)-ether

Die Acetalisierung von 0,5 Mol 2-Methylbutyraldehyd mit 1 Mol Norborn-3-en-1-methanol und die daran anschließende Herstellung des Enolethers wurde wie in Beispiel 1 beschrieben durchgeführt. Nach Abnahme eines Vorlaufs destilliert das gewünschte Produkt bei 98-112° C/12 Torr über.

**Beispiel 9**

2-Methylprop-1-enylbenzylether

Die Acetalisierung von 0,5 Mol Isobutyraldehyd mit 1 Mol Benzylalkohol und die daran anschließende Herstellung des Enolethers wurde wie in Beispiel 1 beschrieben durchgeführt. Das gewünschte Produkt destilliert nach Spaltung des Acetals bei ca. 43° C/10 Torr über.

**Beispiel 10**

2-Ethylbut-1-enylbenzylether

Die Acetalisierung von 0,5 Mol 2 Ethylbutyraldehyd mit 1 Mol Benzylalkohol und die daran anschließende Herstellung des Enolethers wurde wie in Beispiel 1 beschrieben durchgeführt. Das gewünschte Produkt destilliert nach Spaltung des Acetals bei ca. 120° C/10 Torr über.

**Beispiel 11**

Folgende Kautschukmischung wurde auf der Walze hergestellt:

| | |
|---|---|
| Naturkautschuk | 100,0 Gew.-Teile |
| Zinkoxid | 10,0 Gew.-Teile |
| Gefällte Kreide | 160,0 Gew.-Teile |
| Titandioxid | 10,0 Gew.-Teile |
| Stearinsäure | 0,7 Gew.-Teile |
| Ozonschutzwachs | 2,0 Gew.-Teile |
| Dibenzothiazyldisulfid | 1,0 Gew.-Teile |
| Hexamethylentetramin | 0,25 Gew.-Teile |
| Schwefel | 2,2 Gew.-Teile |
| Ozonschutzmittel | 4,0 Gew.-Teile |

Von diesen Mischungen wurden Prüfkörper von 0,4 x 4,5 x 4,5 cm vulkanisiert (Preßvulkanisation (30 min bei 150° C)). Je 4 Prüfkörper wurden dann in einem Kunststoffrahmen so eingespannt, daß an der Oberfläche Dehnungen von 10, 20, 30 und 60 % entstehen.

Die gespannten Prüfkörper wurden mit einem Luftstrom, der 100 Teile Ozon auf 100 Millionen Teile Luft enthielt, bei Raumtemperatur behandelt. Nach jeweils 2, 4, 6, 8, 24, 48, 72, 96, und 168 Stunden wurden die Proben mit dem Auge auf eventuelle Risse untersucht. In der nachfolgenden Tabelle sind jewells die Stunden bis zur ersten Rißbildung eingetragen. Nach 168 Stunden wurden die Versuche abgebrochen (Tabelle 1).

**Beispiel 12**

Folgende Kautschukmischung wurde auf der Walze hergestellt:

| | |
|---|---|
| Styrol-Butadien-Mischpolymerisat | 100,0 Gew.-Teile |
| Zinkoxid | 5,0 Gew.-Teile |
| Ruß (N 220) | 55,0 Gew.-Teile |
| Naphthtenischer Mineralölweichmacher | 2,0 Gew.-Teile |
| Hocharomatischer Mineralölweichmacher | 2,0 Gew.-Teile |
| Stearinsäure | 2,0 Gew.-Teile |

| | |
|---|---|
| Ozonschutzwachs | 2,0 Gew.-Teile |
| Benzothiazyl-2-cyclohexylsulfenamid | 1,3 Gew.-Teile |
| Schwefel | 1,6 Gew.-Teile |
| Ozonschutzmittel | 4,0 Gew.-Teile |

Die Prüfkörper wurden in der Presse 30 Min. bei 150° C vulkanisiert. Die Prüfung wurde ebenso durchgeführt wie in Beispiel 11 beschrieben, allerdings betrug die Ozonkonzentration statt 100 Teile jetzt 200 Teile pro 100 Millionen Teile Luft (Tabelle 2).

**Tabelle 1** Naturkautschuk (NR)

| Dehnung (%) | 10 | 20 | 30 | 60 |
|---|---|---|---|---|
| DE-OS 2 548 911, Bsp. 7 (Vergleich) | 48 | 2 | 2 | 2 |
| Produkt n. Bsp. 4 | 72 | 24 | 4 | 2 |
| Produkt n. Bsp. 3 | 72 | 24 | 8 | 6 |
| Produkt n. Bsp. 2 | >168 | >168 | >168 | >168 |
| Produkt n. Bsp. 1 | >168 | >168 | >168 | >168 |
| Produkt n. Bsp. 5 | >168 | >168 | >168 | >168 |
| Produkt n. Bsp. 6 | >168 | 96 | 24 | 24 |
| Produkt n. Bsp. 7 | >168 | >168 | >168 | >168 |
| Produkt n. Bsp. 8 | >168 | >168 | >168 | >168 |
| Produkt n. Bsp. 9 | >168 | >168 | 4 | 4 |
| Produkt n. Bsp.10 | >168 | >168 | >168 | >168 |

**Tabelle 2** Styrol-Butadien-Kautschuk (SBR)

| Dehnung (%) | 10 | 20 | 30 | 60 |
|---|---|---|---|---|
| DE-OS 2 548 911, Bsp. 7 | >168 | >168 | 8 | 2 |
| Produkt n. Bsp. 2 | >168 | >168 | >168 | 24 |
| Produkt n. Bsp. 1 | >168 | >168 | >168 | >168 |
| Produkt n. Bsp. 5 | >168 | >168 | >168 | >168 |
| Produkt n. Bsp. 6 | >168 | >168 | 72 | 48 |
| Produkt n. Bsp. 7 | >168 | >168 | >168 | >168 |
| Produkt n. Bsp. 8 | >168 | >168 | 72 | 48 |
| Produkt n. Bsp. 9 | >168 | >168 | 48 | 48 |
| Produkt n. Bsp.10 | >168 | >168 | >168 | 96 |

**Patentansprüche**

1. Verfahren zur Verbesserung der Ozonbeständigkeit von Natur- und/oder Synthesekautschuk, dadurch gekennzeichnet, daß Enolether der Formel I

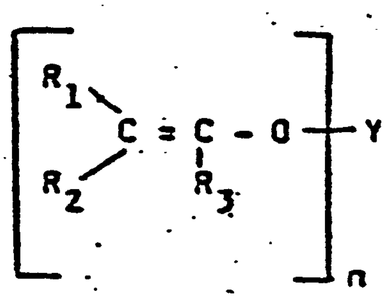

in der

n 1 oder 2,

$R^1$ $C_1$-$C_{10}$-Alkyl oder $C_2$-$C_{10}$-Alkenyl, welche gegebenenfalls ein- oder mehrfach, bevorzugt ein- bis dreifach durch Chlor oder $C_1$-$C_4$-Alkyl substituiert sind, $C_5$-$C_{20}$-Cyclo- oder Bicycloalkyl oder -alkenyl; $C_6$-$C_{10}$-Aryl, gegebenenfalls ein- oder mehrfach, bevorzugt ein- bis fünffach durch Chlor und/oder $C_1$-$C_4$-Alkyl substituiert oder $C_6$-$C_{10}$-Aryl-$C_1$-$C_8$-alkyl, wobei die Arylgruppe ein- oder mehrfach, bevorzugt ein- bis fünffach durch Halogen und/oder $C_1$-$C_4$-Al-kyl substituiert sein kann, bedeutet,

$R^2$ und $R^3$ Wasserstoff bedeuten oder die Bedeutung von $R^1$ aufweisen und die beiden Substituenten $R_1$ und $R_3$ zu einem mindestens fünfgliedrigen Ring miteinander verbunden sein können, und

Y im Fall von n = 1

$C_5$-$C_7$-Cycloalkyl, $C_5$-$C_7$- Bicycloalkyl, $C_5$-$C_7$-Cycloalkenyl, $C_5$-$C_7$-Bicycloalkenyl, $C_6$-$C_{10}$-Aryl, $C_5$-$C_7$-Cycloalkyl-$C_1$-$C_4$-alkyl, $C_5$-$C_7$-Cycloalkenyl-$C_1$-$C_4$-alkyl, $C_5$-$C_7$-Bicycloalkenyl-$C_1$-$C_4$-alkyl oder gegebenenfalls durch ein bis drei Sauerstoff- oder durch $R_4$ substituierte Stickstoffatome unterbrochenes $C_4$-$C_{12}$ Alkyl und $C_4$-$C_{12}$-Alkenyl,

im Fall n = 2

$C_5$-$C_7$ Cycloalkylen, $C_6$-$C_{10}$ Arylen, Xylylen Hexahydroxylylen oder gegebenenfalls durch ein bis drei Sauerstoff- oder durch $R_4$ substituierte Stickstoffatome unterbrochenes $C_4$-$C_{12}$ Alkylen darstellt und wobei alle Reste Y gegebenenfalls ein- oder mehrfach, bevorzugt ein- bis vierfach durch Chlor substituiert sind und

$R_4$ $C_1$-$C_4$-Alkyl bedeutet,

in Kautschuk eingearbeitet werden.

2. Verfahren gemäß Anspruch 1, wobei

$R_1$ $C_1$-$C_6$-Alkyl

$R_2$ Wasserstoff, Methyl oder Ethyl,

$R_3$ Wasserstoff und

Y Tetramethylen, Hexamethylen, Dihexamethylenether, Xylylen, 1,4-Dimethylencyclohexylen, $C_4$-$C_{10}$-Alkyl, $C_5$-$C_7$-Cycloalkenylmethyl oder $C_5$-$C_7$-Bicycloalkenylmethyl

darstellen.

3. Verbindungen der Formel

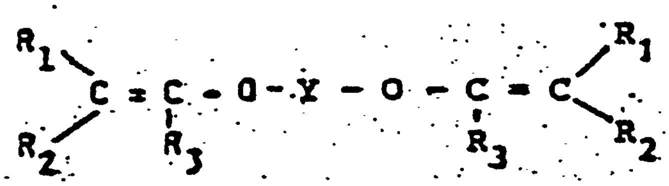

worin $R_1$, $R_2$ und $R_3$ die in Anspruch 1 genannte Bedeutung besitzen und

Y die Bedeutungen $C_5$-$C_7$-Cycloalkylen, $C_6$-$C_{10}$-Arylen, Xylylen oder gegebenenfalls durch ein bis drei Sauerstoffatome oder durch $NR_4$-Gruppen unterbrochenes $C_4$-$C_{12}$-Alkylen hat und Y gegebenenfalls ein- oder mehrfach durch Chlor substituiert sein kann und

$R_4$ $C_1$-$C_6$-Alkyl bedeutet.

4. Verbindungen nach Anspruch 3, worin

$R_1$ $C_1$-$C_6$-Alkyl

$R_2$ Wasserstoff, Methyl oder Ethyl oder $R_1$ und $R_2$ zusammen Pentamethylen,

$R_3$ Wasserstoff und

Y Tetramethylen, Hexamethylen, Di-hexamethylenether, Xylylen oder 1,4-Dimethylencyclohexylen bedeuten.

## Claims

1. Process for inproving the ozone resistance of natural and/or synthetic rubber, characterised in that enol ethers of the formula I

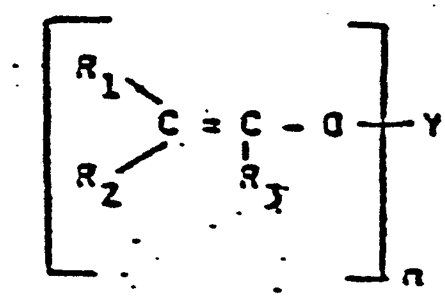

in which
n denotes 1 or 2,
$R^1$ denotes $C_1$-$C_{10}$-alkyl or $C_2$-$C_{10}$-alkenyl, which are optionally mono- or polysubstituted, preferably mono- to trisubstituted by chlorine or $C_1$-$C_4$-alkyl, $C_5$-$C_{20}$-cyclo- or bicycloalkyl or -alkenyl; $C_6$-$C_{10}$-aryl, optionally mono- or polysubstituted, preferably mono- to pentasubstituted by chlorine and/or $C_1$-$C_4$-alkyl or $C_6$-$C_{10}$-aryl-$C_1$-$C_8$-alkyl, it being possible for the aryl group to be mono- or polysubstituted, preferably mono- to pentasubstituted by halogen and/or $C_1$-$C_4$-alkyl,
$R^2$ and $R^3$ denote hydrogen or have the meaning of $R^1$ and the two substituents $R_1$ and $R_3$ can be linked to each other to form an at least five-membered ring, and,
in the case where n = 1, Y represents $C_5$-$C_7$-cycloalkyl, $C_5$-$C_7$-bicycloalkyl, $C_5$-$C_7$-cycloalkenyl, $C_5$-$C_7$-bicycloalkenyl, $C_6$-$C_{10}$-aryl, $C_5$-$C_7$-cycloalkyl-$C_1$-$C_4$-alkyl, $C_5$-$C_7$-cycloalkenyl-$C_1$-$C_4$-alkyl, $C_5$-$C_7$-bicycloalkenyl-$C_1$-$C_4$-alkyl or $C_4$-$C_{12}$-alkenyl and $C_4$-$C_{12}$-alkyl, optionally interrupted by one to three oxygen atoms or by nitrogen atoms substituted by $R_4$, or
in the case where n = 2, Y represents $C_5$-$C_7$-cycloalkylene, $C_6$-$C_{10}$-arylene, xylylene hexahydroxylylene or $C_C$-$C_{12}$-alkylene which is optionally interrupted by one to three oxygen atoms or by nitrogen atoms substituted by $R_4$, and wherein all of the radicals Y are optionally mono- or polysubstituted, preferably mono- to tetrasubstituted by chlorine and
$R_4$ denotes $C_1$-$C_4$-alkyl,
are incorporated into rubber.

2. Process according to Claim 1, wherein
$R_1$ represents $C_1$-$C_6$-alkyl,
$R_2$ represents hydrogen, methyl or ethyl,
$R_3$ represents hydrogen and
Y represents tetramethylene, hexamethylene, dihexamethylene ether, xylylene, 1,4-dimethylene-cyclohexylene, $C_4$-$C_{10}$-alkyl, $C_5$-$C_7$-cycloalkenylmethyl or $C_5$-$C_7$-bicycloalkenylmethyl.

3. Compounds of the formula

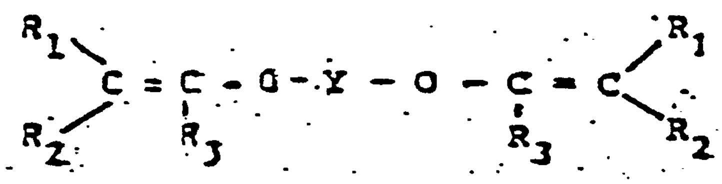

wherein
$R_1$, $R_2$ and $R_3$ have the meaning given in Claim 1 and
Y has the meanings $C_5$-$C_7$-cycloalkylene, $C_6$-$C_{10}$-arylene, xylylene or $C_4$-$C_{12}$-alkylene which is optionally interrupted by one to three oxygen atoms or by $NR_4$ groups and Y can be optionally mono- or polysubstituted by chlorine and
$R_4$ denotes $C_1$-$C_6$-alkyl.

4. Compounds according to Claim 3, wherein

$R_1$ denotes $C_1$-$C_6$-alkyl,
$R_2$ denotes hydrogen, methyl or ethyl or $R_1$ and $R_2$ together denote pentamethylene,
$R_3$ denotes hydrogen and
Y denotes tetramethylene, hexamethylene dihexamethylene ether, xylylene or 1,4-dimethylenecyclohexylene.

## Revendications

1. Procédé pour l'amélioration de la stabilité à l'ozone du caoutchouc naturel et/ou du caoutchouc synthétique, caractérisé en ce qu'on incorpore dans le caoutchouc des éthers énoliques de formule générale (I)

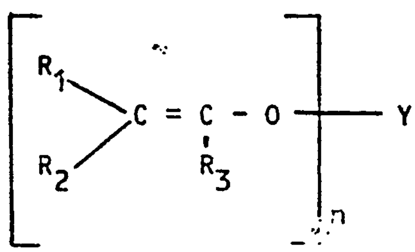

(I)

dans laquelle
n est égal à 1 ou 2,
$R^1$ représente un groupe alkyle en $C_1$-$C_{10}$ ou alcényle en $C_2$-$C_{10}$, qui sont éventuellement substitués une ou plusieurs fois, de préférence une à trois fois, par le chlore ou des groupes alkyles en $C_1$-$C_4$; un groupe cyclo- ou bicyclo-alkyle ou -alcényle en $C_5$-$C_{20}$; un groupe aryle en $C_6$-$C_{10}$, éventuellement substitué une ou plusieurs fois, de préférence une à cinq fois, par le chlore et/ou des restes alkyles en $C_1$-$C_4$, ou un groupe (aryl en $C_6$-$C_{10}$)-alkyle en $C_1$-$C_8$, le groupe aryle pouvant être substitué une ou plusieurs fois, de préférence une à cinq fois, par un halogène et/ou par un groupe alkyle en $C_1$-$C_4$,
$R^2$ et $R^3$ représentent l'hydrogène ou possèdent la signification de $R^1$, et
les deux substituants $R^1$ et $R^3$ peuvent être reliés ensemble en un cycle à au moins cinq chaîns, et
dans le cas où n = 1,
Y représente un groupe cycloalkyle en $C_5$-$C_7$, bicycloalkyle en $C_5$-$C_7$, cycloalcényle en $C_5$-$C_7$, bicycloalcényle en $C_5$-$C_7$, aryle en $C_6$-$C_{10}$, (cycloalkyl en $C_5$-$C_7$)-alkyle en $C_1$-$C_4$, (cycloalcényl en $C_5$-$C_7$)-alkyle en $C_1$-$C_4$, (bicycloalcényl en $C_5$-$C_7$)-alkyle en $C_1$-$C_4$ ou un groupe alkyle en $C_4$-$C_{12}$ ou alcényle en $C_4$-$C_{12}$, éventuellement interrompu par 1 à 3 atomes d'oxygène ou atomes d'azote substitués par $R_4$,
dans le cas où n = 2,
Y représente un groupe cycloalkylène en $C_5$-$C_7$, arylène en $C_6$-$C_{10}$, xylylène, hexahydroxylylène ou un groupe alkylène en $C_4$-$C_{12}$, éventuellement interrompu par 1 à 3 atomes d'oxygène ou atomes d'azote substitués par $R_4$, et
tous les restes Y étant éventuellement substitués une ou plusieurs fois, de préférence 1 à 4 fois, par le chlore et $R_4$ représente un groupe alkyle en $C_1$-$C_4$.

2. Procédé selon la revendication 1, caractérisé en ce que:
$R_1$ est un groupe alkyle en $C_1$-$C_6$,
$R_2$ est l'hydrogène ou un groupe méthyle ou éthyle,
$R_3$ est l'hydrogène et
Y est un groupe tétraméthylène, hexaméthylène, 6,6'-oxy-bis-hexyle, xylylène, cyclohexylène-1,4-diméthyle, alkyle en $C_4$-$C_{10}$, (cycloalcényl en $C_5$-$C_7$)-méthyle, (bicycloalcényl en $C_5$-$C_7$)-méthyle.

3. Composés de formule

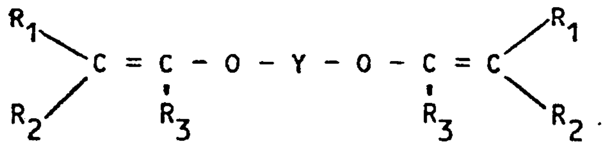

dans laquelle $R_1$, $R_2$ et $R_3$ ont les significations indiquées à la revendication 1 et

Y représente un groupe cycloalkylène en $C_5$-$C_7$, arylène en $C_6$-$C_{10}$, xylylène, ou alkylène en $C_4$-$C_{12}$ éventuellement interrompu par 1 à 3 atomes d'oxygène ou groupes $NR_4$ et Y peut être éventuellement substitué une ou plusieurs fois par le chlore, et

$R_4$ représente un groupe alkyle en $C_1$-$C_6$.

4. Composés selon la revendication 1, caractérisés en ce que:

$R_1$ représente un groupe alkyle en $C_1$-$C_6$,

$R_2$ représente l'hydrogène ou un groupe méthyle ou éthyle, ou bien $R_1$ et $R_2$, pris ensemble, représentent un groupe pentaméthylène,

$R_3$ représente l'hydrogène, et

Y représente un groupe tétraméthylène, hexaméthylène, 6,6'-oxy-bis-hexyle, xylylène ou cyclohexylène-1,4-diméthyle.